(19) Europäisches Patentamt
European Patent Office
Office européen des brevets

(11) Publication number: **0 294 944 B1**

# (12) EUROPEAN PATENT SPECIFICATION

(45) Date of publication of patent specification: **05.08.92**

(51) Int. Cl.⁵: **C10M 159/20**, C10M 159/22, C10M 129/54, C10M 145/18, C10M 145/20, C07C 65/105, //(C10N30/04,40:00)

(21) Application number: **88304332.5**

(22) Date of filing: **13.05.88**

(54) **Lubricant compositions and method for preparation of same.**

(30) Priority: **12.06.87 JP 146434/87**

(43) Date of publication of application: **14.12.88 Bulletin 88/50**

(45) Publication of the grant of the patent: **05.08.92 Bulletin 92/32**

(84) Designated Contracting States: **DE FR GB NL**

(56) References cited:
**EP-A- 0 015 761**
**WO-A-88/04685**
**GB-A- 734 622**
**US-A- 3 704 315**
**US-A- 3 793 201**
**EP-A- 0 022 701**
**DE-A- 1 963 046**
**US-A- 2 253 811**
**US-A- 3 766 067**

(73) Proprietor: **TAIYO CHEMICAL CO. LTD.**
**12-1, Nihonbashi Kabuto-cho Chuo-ku**
**Tokyo(JP)**

(72) Inventor: **Murakami, Mitsuhiro**
**515 Nishi Tanaka**
**Gotemba-shi Shizuoka-ken(JP)**
Inventor: **Mikami, Takahiro**
**Chuoh Green Town, 767-7 Hagiwara**
**Gotemba-shi Shizuoka-ken(JP)**
Inventor: **Tokuno, Mitsuji**
**213-18 Nagatsuka**
**Gotemba-shi Shizuoka-ken(JP)**
Inventor: **Nagamatsu, Hiroyuki**
**1289-578 Niihashi**
**Gotemba-shi Shizuoka-ken(JP)**

(74) Representative: **Curtis, Philip Anthony et al**
**Eric Potter & Clarkson St. Mary's Court St. Mary's Gate**
**Nottingham NG1 1LE(GB)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

The present invention relates to a lubricant composition and to a method of preparing the same.

The present invention more specifically relates to new and excellent lubricant compositions having improved engine properties such as detergency/dispersancy, thermal stability, hydrolytic stability, oil solubility, and especially compatibility with other additives. More particularly, the present invention relates to lubricant compositions comprising one or more lubricants and polyvalent metal salt of formaldehyde condensation products of $C_{12}$ - $C_{22}$ alkyl hydroxy benzoic acid, and a method of preparation of same.

Various properties are required for lubricants to be utilized for internal combustion engines. It has come to be common knowledge to add one or more chemical additives to a lubricant in order to improve engine properties. Depending upon the required properties, various additives such as detergents/dispersants, anti-oxidants, antifoam agents, viscosity-index improvers, corrosion inhibitors, rust inhibitors and the like have been utilized. Among them, detergents/dispersants are generally added to various lubricants to be used for internal combustion engines are are particularly important.

In recent years, internal combustion engines have come to be operated continuously for a longer span of time at higher temperatures due to the improvements in the structure and the material of engines. This has created a demand for engine oils which may withstand continuous use for a longer span of time at higher temperatures. Improvements of engine oils in various properties, even though they are small in numerical value, have great significance from a practical view point.

Various properties of engine oils are derived from the chemical structures of the additives and the functional groups contained therein. Different chemical structures may give different properties or characteristics and the increase in kind and number of the functional groups included may give different activities. Also it is true that as the inclusion of functional groups in a molecule of an additive compound increases in kind and number, the activities thereof are extended over wider aspects and the effects thereof increase.

A conventional detergent/dispersant typically comprises an oil-soluble metal salt as a surface active agent. It is understood that the cleaning and dispersing activities of detergent/dispersant are derived from polar groups and oleophilic groups included therein; more particularly the polar groups adsorb insolubles produced in engines during their operation and oleophilic groups disperse the insolubles adsorbed thereto into oils. Typical polar groups are the hydroxyl group, the sulfonic acid group, the carboxylic acid group, and the phosphoric acid group. Classification of detergents/dispersants is usually based on the kind of polar groups included therein. For instance, the so-called metal phenate type additives contain a hydroxyl group attached to phenol, the metal sulphonate type additives contain a sulfonic acid group, the metal phosphate type additives contain a phosphoric acid group, and the metal salicylate type additives contain a hydroxyl group and a carboxylic acid group which are attached to a benzene nucleus at adjacent positions.

As previously mentioned, the uses of lubricants are extended over various aspects because the operational engine mechanism is complicated. Thus the combined use of two or more kinds of additives has often been made, since the use of a single additive which includes less functional groups in kind and number is insufficient for required purposes. In such a case, two or more additives are generally used together in the expectation that they will co-operate. However, there is a problem of whether the additives to be jointly used are soluble in lubricants without affecting with each other. In general, additives must be soluble with each other, and even though each of said additives is soluble in oils, they are not always used jointly. Poor solubility of additives individually or jointly in oils may result turbidity and/or precipitation problems which entail malfunction of engines. For example, conventional additives of calcium salts of alkyl hydroxy benzoic acid and additives of the calcium sulfonate type are insoluble jointly, and they cannot be used jointly because of a precipitation problem.

EP 0022701 discloses poly(alkylene oxide) compositions which contain a small amount of a bridged dimer of a hydroxyl-substituted aromatic carboxylic acid or a salt thereof.

The operational conditions of the engine become more and more severe, and it is desired to provide lubricants which may withstand higher temperature for a longer span of time.

It is often observed that there is an anxiety over the contamination of water in engine oils, and the intermixed water may promote or enhance hydrolysis of additive compounds at high temperatures. Therefore additives for lubricants are required to be stable against hydrolysis at high temperature.

Conventional additives, however, are inadequate in general for the reasons described above.

This problem can be solved by preparing chemical additives which include a plurality of different functional groups, particularly hydroxy benzoic acid, which include, in a molecule, a hydroxyl group and a carboxylic acid group as polar groups. Such additives can be prepared by taking two molecules of alkyl hydroxy benzoic acid, each including $C_{12}$-$C_{22}$ hydrocarbon as an oleophilic group, and condensing them

with formaldehyde.

More precisely, the inventors have found that lubricant compositions which include one or more lubricants and polyvalent metal salt of formaldehyde condensation products of $C_{12}$ - $C_{22}$ alkyl hydroxy benzoic acid show various excellent properties, thermal stability at high temperature, hydrolytic stability, oil solubility and compatibility with other additives, and moreover detergency/dispersancy.

In accordance with one aspect of the invention there is provided a lubricant composition characterised by one or more lubricants and a polyvalent metal salt of formaldehyde condensation products of $C_{12}$ - $C_{22}$ alkyl hydroxy benzoic acid.

The lubricant compositions according to the invention are excellent in detergency/dispersancy at high temperature, thermal stability, hydrolytic stability and oil solubility.

The lubricant compositions according to the invention can be economically prepared without necessity of complicated antipollution measures.

According to the present invention, it is possible to produce lubricant compositions which are excellent in detergency/dispersancy, thermal stability, hydrolytic stability and oil solubility.

As the lubricant compositions of the present invention have many properties in itself, it is possible to reduce the kinds of additives to be jointly used.

Also lubricant compositions of the present invention can be utilized for various type of engine due to their multifunction, including land engines and marine engines.

The active sites on benzene ring which are able to be substituted by alkyl groups in alkylation of phenol are the ortho and/or para positions with respect to the hydroxyl group; the active sites of alkyl phenol for substitution by carboxylic acid groups are the ortho and/or para positions with respect to the hydroxyl group; and the active sites of alkyl hydroxy benzoic acid for substitution by methylene groups of formaldehyde are also ortho and/or para positions with respect to the hydroxyl group.

Therefore, in order to achieve an efficient condensation reaction of alkyl hydroxy benzoic acid with formaldehyde, it is desirable that the alkyl phenol prepared by alkylation of phenol is monoalkyl phenol substituted by only a single alkyl group. If monoalkyl phenol can be efficiently prepared, the condensation reaction of monoalkyl benzoic acid formed from monoalkyl phenol with formaldehyde will be efficiently carried out. Consequently the methylenebis structure aimed by the present invention can be formed by bonding two molecules of alkyl hydroxy benzoic acid with the intervention of a methylene group from formaldehyde.

Thus it will be understood that the polyvalent metal salt of formaldehyde condensation products of alkyl hydroxy benzoic acid prepared in accordance with the present invention contains two hydroxyl groups and two carboxylic acid groups as polar groups and two alkyl groups as oleophilic groups in same molecule. It will be also understood that while a polyvalent metal salt of alkyl hydroxy benzoic acid may show excellent detergency/dispersancy, thermal stability and hydrolytic stability, these properties are further increased by formation of the methylenebis structure according to the present invention, and moreover oil solubility and compatibility with other additives are also increased.

The process of the present invention includes following steps:

(a) a step of preparation of monoalkyl phenol by alkylation of phenol with $C_{12}$ - $C_{22}$ olefin under the presence of an ion exchange resin as the catalyst for alkylation;

(b) a step of preparation of metal phenoxide by addition of caustic alkali to the resulted reaction mixture containing monoalkyl phenol followed by removal of water produced as a byproduct;

(c) a step of carboxylation by reaction of carbon dioxide with the metal phenoxide obtained in step (b) under pressure, followed by hydrolysis with addition of mineral acids to isolate alkyl hydroxy benzoic acid;

(d) a step of condensation of the collected alkyl hydroxy benzoic acid with formaldehyde under the presence of a reaction promoter, followed by isolation and removal of the unreacted formaldehyde and the reaction promoter;

(e) a step of preparation of alkali salt of the resultant condensation products by addition of caustic alkali, followed by conversion into the corresponding polyvalent metal salt by double decomposition with addition of polyvalent metal chlorides;

If occasion demands, a further step described hereunder can be added.

(f) a step of conversion of the resultant polyvalent metal salt into a highly basic salt by addition of an alcoholic suspension of polyvalent metal hydroxides and blowing of carbon dioxide gas thereinto.

The addition of one or more lubricants can be made at the steps 5 and/or 6 in the above.

More specifically, in accordance with the present invention, phenol as the starting substance is subjected to alkylation with an alkylation agent under the presence of catalysts. An olefin with $C_{12}$ - $C_{22}$ - (preferably $C_{14}$ -$C_{18}$) can be utilized as an alkylation agent.

Heretofore, activated clay, metal chlorides, hydrofluoric acid, phosphoric acid and the like have been utilized as the catalysts for alkylation. Above all, activated clay has been widely used for the reasons of easiness in handling and cheapness in cost. However, as activated clay remains in the reaction system, it necessitates the procedures of filtration and disposal and entails necessity of anti-pollution measures. All the other liquid inorganic acids belong to highly dangerous substances, and involve dangers in handling. Recently ion-exchange resins which are developed as solid organic acids and usable as catalysts for alkylation became available in the market. In Japanese Patent Application No. 84/102/59(1984), there is disclosed that highly acidic sulfonated polystyrene type resins are excellent as alkylation catalysts among the acidic ion-exchange resins, that they have longer life cycle, and they can be repetitively utilized. Also they are highly advantageous from the viewpoint of antipollution measures, and most attractively they are suited to prepare predominantly monoalkyl phenol in high yields. As such a catalyst, for example, DIAION RCP-145H (by Mitsubishi Kasei Company) has been sold in the market, and found to be suitable as the alkylation catalyst in the present invention.

For example, to the mixture of 2 - 3 moles of phenol and 1 mole of olefin, DIAION RCP-145H can be added, and the resultant mixture is subjected to alkylation at a temperature between 90 - 150 °C, preferably 110 - 135 °C, for 2 - 3 hours to yield 95 - 98 mol. % of monoalkyl phenols. The quantity of the catalyst to be used ranges between 3 - 10 wt. % of total quantity of phenol and olefin. Yields of monoalkyl phenol in each case wherein DIAION RCP-145H, activated clay and the combination of activated clay and phosphoric acid were utilized are shown in the following table.

| catalyst | DIAION RCP145H | activated clay | activated clay and phosphoric acid |
|---|---|---|---|
| ratio of olefin to phenol | 0.5 | 0.5 | 0.5 |
| quantity of catalyst (wt.%) | 4 | 6 | 3 |
| reaction temperature (°C) | 100 | 160 | 120 |
| reaction hours | 2 | 6 | 3 |
| yield of mono-alkylphenols (mole %) | 97 | 75 | 80 |

The resultant monoalkyl phenol is converted into alkyl hydroxy benzoic acid via metal phenoxide by the well known Kolbe-Schmitt reaction. For example, to 1 mole of alkyl phenol, 1 mole of caustic alkali is added, the resultant mixture is subjected to reaction at 60 - 65 °C for about 1 hour, then the temperature of the reacted mixture is elevated to 200 °C to remove the byproduct water. The resultant reaction mixture is poured into an autoclave, and 1.5 - 2.2 moles of carbon dioxide gas is blown into said mixture under 5 - 15 atmospheres (507 to 1520 KPa) at 140 - 180 °C for 1 - 3 hours. After the reaction, 30 - 50% of sulfuric acid is added for hydrolysis and the resultant alkyl hydroxy benzoic acid is isolated and collected.

The resultant alkyl hydroxy benzoic acid is subjected to a condensation reaction with formaldehyde under the presence of concentrated sulfuric acid as reaction promoter. Formalin available in the market is adequate for handling and can be utilized as formaldehyde.

For example, to 1 mole of alkyl hydroxy benzoic acid, 20 - 50 wt. % of concentrated sulfuric acid and 0.5 - 4 moles of formaldehyde are added and the resultant mixture is subjected to a condensation reaction at 20 - 100 °C for about 3 - 5 hours. During the condensation reaction, aliphatic hydrocarbon such as hexane and heptane, which do not participate or contribute to the reaction, can be added. After the

condensation reaction, unreacted formaldehyde and sulfuric acid are isolated and removed from the mixture, and the remaining mixture is washed with water sufficiently. For complete isolation, it is preferable to dilute the reaction mixture with aromatic hydrocarbon such as benzene and toluene. Thus the condensation products of alkyl hydroxy benzoic acids with formaldehyde can be obtained.

To the resultant condensation products, mineral oil is added to prepare 100 - 180 wt.% mineral oil solution of said condensation products. To the resultant solution, caustic alkali is added to convert the resultant condensation products into the corresponding alkali salt. To the resultant solution, methanol solution of polyvalent metal chloride is added, and the resultant mixture is kept at the reflux temperature of methanol for about 1 hour, and then the temperature of the solution is elevated to higher than 110 °C to remove volatile substances, to thereby obtain a mineral oil solution of the polyvalent metal salt of formaldehyde condensation products of alkyl hydroxy benzoic acid.

Though any of magnesium, calcium, strontium and barium can be utilized as polyvalent metals, calcium is preferable.

When the lubricant composition prepared in accordance with the present invention is utilized as an additive for lubricants, it is preferable to use it in the form of basic salts or highly basic salts. Basic or highly basic salts can be prepared by adding a methanol suspension of polyvalent metal hydroxide to the resultant polyvalent metal salt of the formaldehyde condensation products of alkyl hydroxy benzoic acid, and to the resultant mixture carbon dioxide is reacted at 20 - 30 °C. The quantities of polyvalent metal hydroxide and carbon dioxide to be used are determined according to the desired base number.

The term "basic" is determined by the excessive amount of polyvalent metal in a unit weight of the polyvalent metal salt of the condensation product compared to that in the corresponding neutral salt, and is represented with total base numbers (TBN). That is, the basicity is represented by the number of milligrams of KOH corresponding to the equivalent quantity of acid which is required to neutralize the total alkaline content in 1 g of the salt. In general, lubricant additives are used in highly basic state for the additional purpose of neutralizing acids produced in engines. The degree of the highly basic state is also represented by the term of metal ratio which is determined by the following formula:

metal ratio = (equivalent of metal/equivalent of organic acid) - 1

Therefore, the metal ratio of a neutral salt is zero. The range of metal ratios of the lubricant compositions in accordance with the present invention is between 0 - 8, and preferably 3 - 5.

The lubricants to be included in the lubricant composition of the present invention may be mineral oils, synthetic lubricants, fatty oils derived from animals and plants, but petroleum lubricants as mineral oil are preferable. The kind and quantity of lubricants to be added may vary depending upon the various purposes, for instance, for the purposes of adjustment of viscosity during the process and of regulation of TBN in production and so on. The lubricant compositions of the present invention can be utilized in conjunction with other additives such as detergents/dispersants, anti-oxidants, viscosity-index improvers, foam inhibitors and so on.

Having described the present invention in general, now some examples will be described hereunder.

EXAMPLES 1 - 6

Monoalkyl phenol was prepared in such a manner that to respective mixtures consisting of a quantity of olefin and a quantity of phenol as shown in Table 1, a quantity of DIAION RCP-145H was respectively added as a catalyst. The resultant mixtures were subjected to an alkylation reaction under agitation at a suitable velocity necessary to eliminate sedimentation of the catalyst at 110 °C for 2 hours, then the reacted mixtures were subjected to vacuum distillation at 5 mm Hg (667 Pa). The quantities of reaction components and yields of monoalkyl phenol are shown in Table 1.

TABLE 1

| Example | phenol (g) | olefin (g) | catalyst (g) | monoalkyl phenol yield (mole %) | carbon atoms included in olefin |
|---|---|---|---|---|---|
| 1 | 280 | 210 | 23.5 | 90 | 10 * |
| 2 | ditto | 252 | 25.5 | 97 | 12 |
| 3 | ditto | 294 | 27.5 | 97 | 14 |
| 4 | ditto | 336 | 29.5 | 98 | 16 |
| 5 | ditto | 346 | 30.0 | 95 | 15 - 20 |
| 6 | ditto | 357 | 30.5 | 96 | 16 - 18 |

* The olefin used in example 1 is out of the range of the present invention.

EXAMPLES 7 - 12

Using the respective monoalkyl phenol prepared in examples 1 - 6, the following processes were carried out under the same conditions. To a quantity of respective monoalkyl phenol, respective quantities are shown in Table 2 of caustic alkali were added under heating to 50° C, the resultant mixtures were reacted at 65° C for 1 hour, then nitrogen gas was blown into the reacted mixtures under gradual elevation of the temperature up to 200° C and maintained at this temperature for 2 hours to remove water produced as a byproduct. After cooling the resultant reaction mixtures, they were poured into autoclaves with stirrers and then carbon dioxide gas was blown into the mixtures at 140° C under 10 atmospheres (1013 KPa) during 2 hours. After cooling the reaction mixtures to room temperature, they were poured into separatory funnels, and 30% sulfuric acid was gradually added thereto under shaking and stirring respectively for neutralization, thereby alkyl hydroxy benzoic acid was respectively separated and collected from the mixtures.

To the respectively collected alkyl hydroxy benzoic acid, the same quantity of hexane and 50 wt.% of concentrated sulfuric acid were respectively added. To the resultant mixtures, respective quantities of formaldehyde were added under agitation, then the resultant mixtures were reacted under a nitrogen gas atmosphere at 65° C for 5 hours. After removing hexane respectively, the residues were diluted with same quantity of xylene respectively. After settling the resultant mixtures, sulfuric acid and unreacted formalin were removed by decantation. From the respective residues, xylene was removed by distillation to thereby obtain respective condensation products. To the resultant condensation products, mineral oil was respectively added to adjust said condensation products to TBN 65 by mineral oil. The resultant solutions were neutralized respectively with sodium hydroxide to form mineral oil solution of the corresponding sodium salt, then respective quantities of potassium chloride dissolved into fourfold quantities of methanol were added thereto. After keeping the resultant mixtures at the reflux temperature of methanol for 1 hour for double decomposition, the temperature of the resultant reaction mixtures were elevated under the blowing of nitrogen gas to remove methanol. After cooling the resultant mixtures to room temperature, they were diluted with same quantity of xylene, then subjected to filtration to remove any sodium chloride produced. To the resultant xylene solutions containing calcium salt of the condensation products of alkyl hydroxy benzoic acid with formalin, respective quantities of slaked lime and fivefold quantities, with respect to the quantities of slaked lime, of methanol were added, and respective quantities of carbon dioxide gas were blown thereinto at 20 - 30° C. Methanol was distilled out of the resultant mixtures, then unreacted slaked lime was removed with filtration, and finally xylene was distilled out of the filtrate. The TBN of the final products are shown in Table 2 together with the respective quantities of the materials used in examples 7 - 12. Also sample names A - F were assigned to the respective products as shown in Table 2 for future

reference in the description hereinafter.

EXAMPLES 13 - 15

Using the monoalkyl phenol prepared in example 2, 4 and 5, mineral oil solutions of alkyl hydroxy benzoic acid were prepared in the same manner as examples 7 - 12. The resultant solutions of said acid were respectively reacted directly with sodium hydroxide to form the corresponding sodium salt without condensation process. The mineral oil solutions of the resultant sodium salt were converted into the corresponding calcium salt with calcium chloride. To the resultant respective calcium salt, slaked lime and methanol were added, then carbon dioxide gas was blown thereinto to obtain mineral oil solutions of highly basic calcium salt. The TBN of the final products are also shown in Table 2, together with the respective quantities of the materials used in examples 13 - 15. Furthermore, sample names G - I were assigned to the respective final products as shown in Table 2 for reference in the descriptions hereinafter.

TABLE 2

| example | alkyl phenol (g) | KOH (g) | $CO_2$ (g) | formalin (g) | $CaCl_2$ (g) | slaked lime (g) | $CO_2$ (g) | final products TBN | smpl.name |
|---|---|---|---|---|---|---|---|---|---|
| 7 | 200 | 56.1 | 75.2 | 256 | 59.5 | 85.5 | 60.7 | 175 | A* |
| 8 | ditto | 50.0 | 67.2 | 229 | 53.1 | 76.3 | 54.2 | 170 | B |
| 9 | ditto | 45.3 | 60.7 | 207 | 40.8 | 69.0 | 49.0 | 175 | C |
| 10 | ditto | 41.3 | 55.4 | 187 | 43.8 | 62.9 | 44.7 | 180 | D |
| 11 | ditto | 40.4 | 54.2 | 185 | 42.8 | 61.5 | 43.7 | 172 | E |
| 12 | ditto | 39.5 | 53.0 | 181 | 41.9 | 60.2 | 42.8 | 177 | F |
| 13 | ditto | 50.0 | 67.2 | - | 53.1 | 76.3 | 54.2 | 173 | G* |
| 14 | ditto | 41.3 | 55.4 | - | 43.8 | 62.9 | 44.7 | 174 | H* |
| 15 | ditto | 40.4 | 54.2 | - | 42.8 | 61.5 | 43.7 | 178 | I* |

* The samples A, G, H and I are not included in the scope of the present invention.

With respect to the samples A - I, engine property tests were conducted as follows:

1) ENGINE TEST (piston cleaning test)

To 9 lots of a paraffinic mineral oil, respective samples were added to prepare 9 kinds of test oils of SAE 30, TBN 7. To the respective test oils, 0.6 wt.% of anti-oxidant (dialkyl zinc thiophosphate) was respectively added. Using a diesel engine (by YANMAR, NSA-40C), engine properties of the test oils were tested.

SPECIFICATION OF THE ENGINE

| | |
|---|---|
| type: | 4 cycle, horizontal, water-cooled |
| maximum power: | 5 PS |
| velocity: | 2400 r.p.m. |
| bore: | 70 mm |
| stroke: | 70 mm |

TEST CONDITION

| | |
|---|---|
| power: | 3.5 PS |
| velocity: | 2200 r.p.m. |
| temperature of crank case water: | 90 ° C |
| test period: | 100 hours |
| fuel: | gas oil (containing 0.5 wt.% sulfur) |
| fuel consumption: | 650 - 750 mℓ/h |

EVALUATION

The quantities and qualities of sludge and lacquer stuck on pistons were observed, and the appearance was evaluated by demerit mark method, representing the best condition as 10.

The results are shown in Table 3

TABLE 3

| sample | A | B | C | D | E | F | G | H | I |
|---|---|---|---|---|---|---|---|---|---|
| evaluation | 8.7 | 9.0 | 9.3 | 9.2 | 9.2 | 9.2 | 8.6 | 8.5 | 8.8 |

2) PANEL COKER TEST

To 9 lots of a paraffinic mineral oil, respective samples were added to prepare 9 kinds of test oils having the values of SAE 30 and TBN 7. Used panels were made of duralumin.

TEST CONDITIONS

| | |
|---|---|
| temperature of panel: | 300 ° C |
| temperatures of oil: | 110 ° C |
| test period: | 3 hours |
| splash condition: | splashing 15 sec./posing 60 sec. |

The results are shown in Table 4.

TABLE 4

| samples | A | B | C | D | E | F | G | H | I |
|---|---|---|---|---|---|---|---|---|---|
| accumulation | 6.3 | 5.0 | 8.0 | 9.7 | 9.9 | 2.0 | 24.5 | 40.0 | 31.1 |

unit: mg

3) OXIDATION STABILITY TEST

To 9 lots of a paraffinic mineral oil, respective samples were added to prepare 9 kinds of test oils having the values of SAE 30 and TBN 7. The tests were carried out in accordance with JIS 2514, at 165.5 ° C for 48 hours. The viscosity increasing ratio at 40 ° C and the increasing tendency of total acidity (mg KOH/g) were observed.

The results are shown in Table 5.

TABLE 5

| samples | A | B | C | D | E | F | G | H | I |
|---|---|---|---|---|---|---|---|---|---|
| viscosity ratio | 1.20 | 1.18 | 1.14 | 1.14 | 1.15 | 1.12 | 1.27 | 1.30 | 1.25 |
| increase of acidity | -0.35 | -0.30 | -0.80 | -0.45 | -0.50 | -0.90 | 0.30 | 0.36 | 0.25 |

4) HYDROLYTIC STABILITY TEST

To 9 lots of a paraffinic mineral oil, respective samples were added to prepare 9 kinds of test oils of SAE 30 and TBN 7. To the respective test oils, 0.6 wt.% of antioxidant (dialkyl zinc thiophosphate) was respectively added. The test was carried out in accordance with modified ASTM D 3619, in such a manner that 100 g of respective test oils were respectively poured into bottles together with 5 g of water, and they were sealed. The sealed bottles were rotated upside down at the velocity of 5 r.p.m. at 93°C for 24 hours to make contents deteriorate. The deteriorated test oils were centrifuged at 12,000 r.p.m. for 1 hour. The retaining TBN ratio of the respective supernatants were measured. The results are shown in Table 6.

TABLE 6

| samples | A | B | C | D | E | F | G | H | I |
|---|---|---|---|---|---|---|---|---|---|
| TBN retention (%) | 88 | 90 | 95 | 95 | 93 | 90 | 83 | 88 | 86 |

5) COMPATIBILITY TEST WITH SULFONATE ADDITIVE

To 9 lots of a paraffinic mineral oil, respective samples and a sulfonate type additive were added in the ratio of 1 : 1 to prepare 9 kinds of test oils of SAE 30 and TBN 20 (0.6 wt.% of antioxidant was respectively added). The viscosity index of said mineral oil was 106 (for severe tests). The test oils were kept 60 days applying the cyclic test conditions, initially at 60°C for 8 hours followed by at 5°C for 16 hours. Successive deterioration was observed with the naked eye. The results are shown in Table 7. Evaluation was made by following three stages evaluation criterion.

THREE STAGES EVALUATION CRITERION

a) - : substantially transparent,
b) ± : foggy
c) + : apparently observed turbidity

TABLE 7

| samples | A | B | C | D | E | F | G | H | I |
|---|---|---|---|---|---|---|---|---|---|
| after 60 days | ± | - | - | - | - | - | + | + | + |

It should be noted that samples A and G - I are comparative samples which are not included within the scope of the present invention.

It will be understood as the characteristics of the present invention that monoalkyl phenol can be used as starting substance, that the use of monoalkyl phenol enables one to carry out the condensation reaction efficiently and that the methylenebis structure enables one to increase the kind and number of functional groups contained in a molecule.

It will be also understood that the lubricant compositions in accordance with the present invention have desirable properties in various aspects as explained in the foregoing tests and that the excellent lubricant compositions can be prepared efficiently and economically without the necessity of anti-pollution measures.

## Claims

1. A lubricant composition characterised by comprising one or more lubricants and a compound having the structural formula:

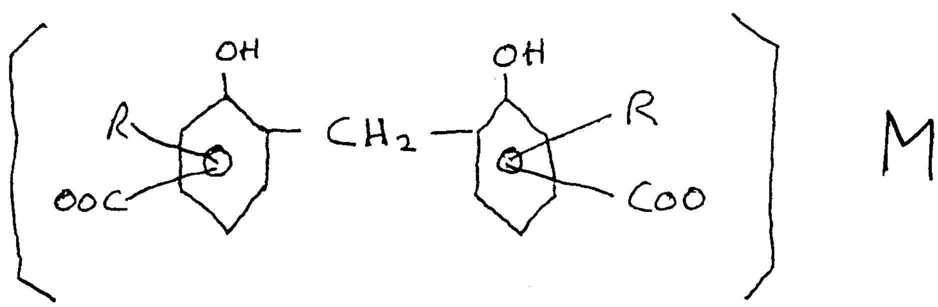

where R is a $C_{12}$ - $C_{22}$ alkyl group and M is a polyvalent metal.

2. A lubricant composition according to Claim 1, characterised in that R is a $C_{14}$ to $C_{18}$ alkyl group.

3. A lubricant composition according to Claim 1 or 2, characterised in that M is calcium.

4. A method for the preparation of lubricant compositions including one or more lubricants and a polyvalent metal salt of formaldehyde condensation products of $C_{12}$ - $C_{22}$ alkyl hydroxy benzoic acid, which method is characterised by:

a) a step of preparation of monoalkyl phenol by alkylation of phenol with $C_{12}$ - $C_{22}$ olefin under the presence of an ion-exchange resin as the catalyst for alkylation;

b) a step of preparation of metal phenoxide by the addition of caustic alkali to the resultant reaction mixture containing monoalkyl phenol followed by removal of water produced as a byproduct;

c) a step of carboxylation by reaction of carbon dioxide with the metal phenoxide obtained in step (b) under pressure, followed by hydrolysis with addition of mineral acids to isolate and collect alkyl hydroxy benzoic acid;

d) a step of condensation of the collected alkyl hydroxy benzoic acid with formaldehyde under the presence of concentrated sulphuric acid as a reaction promoter, followed by isolation and removal of unreacted formaldehyde and reaction promoter; and

e) a step of preparation of alkali salt of the formaldehyde condensation products contained in the resultant solution by addition of caustic alkali and one or more lubricants, followed by conversion into the corresponding polyvalent metal salt by double decomposition with addition of polyvalent

metal chlorides.

5. A method according to Claim 4, characterised in that a further step is provided after step (e) of Claim 4 for conversion of the resultant polyvalent metal salt into highly basic salt by the addition of an alcoholic suspension of polyvalent metal hydroxide and by the blowing of carbon dioxide gas into the resultant mixture.

6. A method according to Claim 4 or 5, characterised in that one or more lubricants are added to the solution obtained at the further step of Claim 5.

**Revendications**

1. Composition lubrifiante caractérisée en ce qu'elle comprend un ou plusieurs lubrifiants et un composé de formule développée :

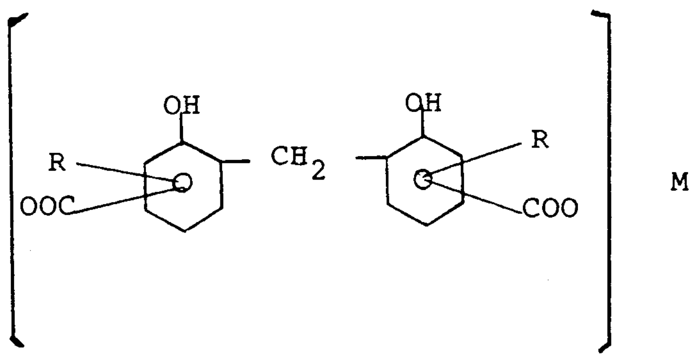

dans laquelle R est un groupe alkyle en $C_{12-22}$ et M est un métal polyvalent.

2. Composition lubrifiante selon la revendication 1, caractérisée en ce que R est un groupe alkyle en $C_{14-18}$.

3. Composition lubrifiante selon la revendication 1 ou 2, caractérisée en ce que M est le calcium.

4. Procédé de préparation de compositions lubrifiantes, y compris un ou plusieurs lubrifiants et un sel métallique polyvalent de produits de condensation de formaldéhyde d'acide alkyle en $C_{12-22}$-hydroxy-benzoïque, lequel procédé se caractérise par :
a) une étape de préparation de mono-alkyl-phénol par alkylation de phénol avec une oléfine en $C_{12-22}$ en présence d'une résine échangeuse d'ions comme catalyseur pour l'alkylation ;
b) une étape de préparation de phénoxyde métallique par l'addition de base caustique au mélange réactionnel résultant contenant du monoalkyl-phénol, suivie par l'enlèvement de l'eau apparue comme sous-produit ;
c) une étape de carboxylation par réaction de dioxyde de carbone avec le phénoxyde métallique obtenu dans l'étape b) sous pression, suivie par une hydrolyse avec addition d'acides minéraux pour isoler et recueillir l'acide alkyl-hydroxy-benzoïque ;
d) une étape de condensation de l'acide alkyl-hydroxy-benzoïque recueilli avec du formaldéhyde en présence d'acide sulfurique concentré comme promoteur de réaction, suivie par un isolement et un enlèvement du formaldéhyde et du promoteur de la réaction n'ayant pas réagis ; et
e) une étape de préparation d'un sel alcalin des produits de condensation de formaldéhyde contenus dans la solution résultante par addition d'une base caustique et d'un ou plusieurs lubrifiants, suivie par une transformation en le sel de métal polyvalent correspondant par un double décomposition avec addition de chlorures de métaux polyvalents.

5. Procédé selon la revendication 4, caractérisé en ce qu'une étape ultérieure est fournie après l'étape e) de la revendication 4 pour la transformation du sel de métal polyvalent résultant en un sel hautement basique par addition d'une suspension alcoolique de l'hydroxyde de métal polyvalent et par l'insufflation de dioxyde de carbone gazeux dans le mélange résultant.

**6.** Procédé selon la revendication 4 ou 5, caractérisé en ce qu'on ajoute un ou plusieurs lubrifiants à la solution obtenue à l'étape ultérieure de la revendication 5.

## Patentansprüche

**1.** Schmiermittelzusammensetzung, dadurch gekennzeichnet, daß sie eine oder mehrere Schmiermittel sowie eine Verbindung mit der Strukturformel

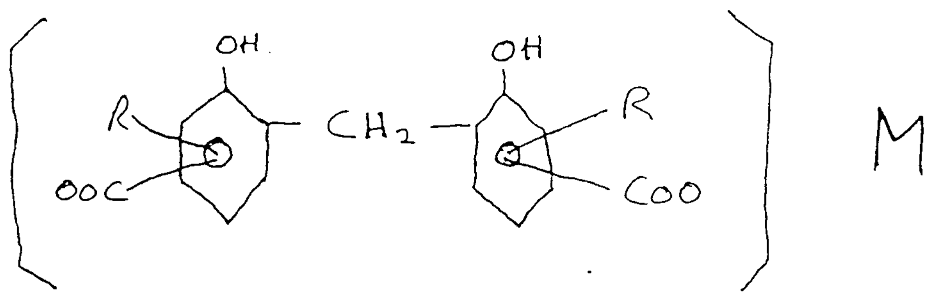

umfaßt, worin R eine $C_{12}$-$C_{22}$-Alkylgruppe und M ein mehrwertiges Metall ist.

**2.** Schmiermittelzusammensetzung nach Anspruch 1, dadurch gekennzeichnet, daß R eine $C_{14}$ bis $C_{18}$-Alkylgruppe ist.

**3.** Schmiermittelzusammensetzung nach Anspruch 1 oder 2, dadurch gekennzeichnet, daß M Calcium ist.

**4.** Verfahren zur Herstellung von Schmiermittelzusammensetzungen, welche eine oder mehrere Schmiermittel und ein mehrwertiges Metallsalz von Formaldehydkondensationsprodukten von $C_{12}$-$C_{22}$-Alkylhydroxybenzoesäure umfaßt, welches Verfahren charakterisiert ist durch:

a) eine Stufe der Herstellung von Monoalkylphenol durch Alkylierung von Phenol mit einem $C_{12}$-$C_{22}$-Olefinin Gegenwart eines Ionenaustauscherharzes als Alkylierungskatalysator;

b) eine Stufe der Herstellung eines Metallphenoxids durch Addition von Alkalihydroxyd zu dem gebildeten Reaktionsgemisch, welches Monoalkylphenol enhält, mit anschließender Entfernung des als Nebenprodukt erzeugten Wassers;

c) eine Stufe der Carboxylierung durch Reaktion von Kohlendioxid mit dem Metallphenoxid, erhalten in der Stufe (b), unter Druck, mit anschließender Hydrolyse durch Addition von Mineralsäuren, zur Isolierung und Gewinnung von Alkylhydroxybenzoesäure;

d) eine Stufe der Kondensation der gesammelten Alkylhydroxybenzoesäure mit Formaldehyd in Gegenwart von konzentrierter Schwefelsäure als ein Reaktionsbeschleuniger mit anschließender Isolierung und Entfernung des nicht umgesetzten Formaldehyds und Reaktionsbeschleunigers; und

e) eine Stufe der Herstellung eines Alkalisalzes der Formaldehydkondensationsprodukte, welche in der gebildeten Lösung enthalten sind, durch Zugabe von Alkalyhydroxyd und einem oder mehreren Schmiermitteln mit anschließender Umwandlung in das entsprechende mehrwertige Metallsalz durch doppelte Zersetzung mit Addition von mehrwertigen Metallchloriden.

**5.** Verfahren nach Anspruch 4, dadurch gekennzeichnet, daß ein weiterer Schritt vorgesehen ist nach der Stufe (e) des Anspruchs 4 zur Umwandlung des gebildeten mehrwertigen Metallsalzes in ein hochbasisches Salz durch Zugabe einer alkoholischen Suspension von mehrwertigem Metallhydroxyd und durch Einblasen von Kohlendioxid in das gebildete Gemisch.

**6.** Verfahren nach Anspruch 4 oder 5, dadurch gekennzeichnet, daß eines oder mehrere Schmiermittel zu der Lösung gegeben werden, welche bei der weiteren Stufe des Anspruchs 5 erhalten wird.